Europäisches Patentamt

(19) European Patent Office

Office européen des brevets

(11) EP 0 587 657 B1

(12) FASCICULE DE BREVET EUROPEEN

(45) Date de publication et mention
de la délivrance du brevet:
**10.03.1999 Bulletin 1999/10**

(21) Numéro de dépôt: **92911326.4**

(22) Date de dépôt: **02.06.1992**

(51) Int. Cl.$^6$: **A23C 9/123**, A23K 1/00,
C12N 1/20
// (C12N1/20, C12R1:01)

(86) Numéro de dépôt international:
**PCT/FR92/00484**

(87) Numéro de publication internationale:
**WO 92/21246 (10.12.1992 Gazette 1992/31)**

(54) **ADDITIF ALIMENTAIRE POUR L'ALIMENTATION HUMAINE ET ANIMALE ET ALIMENTS LE CONTENANT**

NAHRUNGSMITTELZUSATZ ZUR MENSCHLICHEN ERNÄHRUNG UND FÜR FUTTERMITTEL, UND DIESES ENTHALTENDE NAHRUNGSMITTEL

FOOD ADDITIVE FOR HUMAN AND ANIMAL FOODSTUFFS AND FOODSTUFFS CONTAINING SAME

(84) Etats contractants désignés:
**BE DE DK ES GB IT LU NL SE**

(30) Priorité: **03.06.1991 FR 9106641**

(43) Date de publication de la demande:
**23.03.1994 Bulletin 1994/12**

(73) Titulaire: **Florinoval**
**60200 Compiegne (FR)**

(72) Inventeurs:
• **DELESPAUL, Gilbert**
**F-41100 Vendome (FR)**
• **DHOMS, Philippe**
**Areines F-41100 Vendôme (FR)**
• **RAIBAUD, Pierre**
**F-78355 Jouy-en-Josas (FR)**
• **SZYLIT, Odette**
**F-91430 Igny (FR)**

(74) Mandataire:
**Boulinguiez, Didier et al**
**Cabinet Plasseraud**
**84, rue d'Amsterdam**
**75440 Paris Cedex 09 (FR)**

(56) Documents cités:
EP-A- 122 104          EP-A- 190 770
EP-A- 354 828          US-A- 3 262 862

• CHEMICAL ABSTRACTS, vol. 111, no. 17, 23 Octobre 1989, Columbus, Ohio, US; abstract no. 152007F, TANAKA MASAKI: 'CONTINUOUS PRODUCTION OF D-LACTATE BY A HOLLOW FIBER BIOREACTOR' page 565 ;colonne 2 ;
• GHERNA R. ET AL. 'American Type Culture Collection (ATCC)' 1989 , SEVENTEENTH EDITION , ATCC, US, ROCKVILLE
• BIOLOGICAL ABSTRACTS, VOL. 79, Nr.3, 1985 PHILADELPHIA, PA, US; ABSTRACT Nr.21248 KARKI TIKA ET AL.: "MICROORGANISMS ASSOCIATED WITH VARIOUS PICKLES." PAGE AB-326
• JOURNAL OF FOOD SCIENCE. vol. 46, no. 3, 1981, CHICAGO US pages 810 - 812; S. DOORES ET AL.: 'HEAT RESISTANCE OF SPOROLACTOBACILLUS INULINUS'

## Description

La présente invention est relative à un nouvel additif alimentaire microbien, à son application comme biorégulateur aussi bien dans l'alimentation humaine qu'animale ainsi qu'aux aliments le contenant.

De manière générale, au sens de la présente invention, un biorégulateur est un produit qui contribue à l'équilibre digestif et microbien de l'intestin. Ce dernier est essentiellement d'origine microbienne et on peut citer parmi les plus connus ceux dont on a démontré cette activité biorégulatrice :

- des bactéries à Gram + telles que *Lactobacillus, Streptoccocus, Propionibacterium, Bifidobacterium*, certains *Clostridium et Bacillus* ;
- des bactéries à Gram - telle que *Bacteroïdes ;*
- des levures : *Saccharomyces* ;
- des champignons, tels que *Aspergillus.*

Ils sont utilisés, selon les aliments auxquels ils sont incorporés et selon l'effet recherché, seuls ou en association, et dans ce dernier cas, en particulier pour leur effet symbiotique.

On peut citer, à titre d'exemples de biorégulateurs utilisés dans l'alimentation humaine, les microorganismes employés dans les laits fermentés, principalement les bactéries du yaourt ou produits similaires (association de *Lactobacillus bulgaricus*, *Streptococcus thermophilus*, *Laccobacillus acidophilus*, Bifidobactéries, etc...), ou les grains de kéfir (association naturelles de bactéries et de levures) ; on peut citer à titre d'exemples de biorégulateurs utilisés dans l'alimentation animale certains *Bacillus* tels que *Bacillus toyoï*, *Bacillus cereus* ou un mélange symbiotique de *Streptococcus* et *Lactobacillus* tel que décrit dans la Demande de Brevet Européen n° 0 406 117.

De nombreuses hypothèses et mécanismes ont été proposés pour expliquer le rôle biorégulateur de ces microorganismes ; parmi les mécanismes proposés on peut notamment citer :

- l'activité anti-microbienne : ces microorganismes s'opposeraient à la prolifération de bactéries pathogènes en produisant des acides organiques, des antibiotiques, en détoxifiant des entérotoxines bactériennes ou en utilisant la production de métabolites toxiques. Toutefois, le rôle précis de chacun de ces facteurs est difficile à évaluer, car de nombreuses études réalisées *in vitro* sont difficilement transposables au tractus intestinal humain ou animal et actuellement aucune expérimentation humaine ne permet d'affirmer un effet anti-microbien *in vivo*.
- la stimulation du système immunitaire : on a observé que l'absorption régulière des yaourts et par voie de conséquence des souches présentes dans le yaourt (*Lactobacillus bulgaricus, Streptococcus themophillus*) sur des souris axéniques entraînait une augmentation sensible de la quantité d'immunoglobulines G et la présence de *Lactobacillus* vivants dans les ganglions mésentériques.
- l'amélioration de la capacité digestive de l'intestin grêle.
- une induction enzymatique, qui peut avoir deux origines :

    . la β-galactosidase des biorégulateurs hydrolyserait une partie du lactose ingéré au niveau de l'intestin grêle venant ainsi à supplémenter la lactase endogène défaillante,
    . les biorégulateurs ingérés per os stimuleraient la lactase endogène. On a montré qu'un changement de la microflore intestinale serait à l'origine des activités dissacharidiques de l'intestin grêle.

En effet, pour être assimilé, le lactose présent dans la lumière intestinale doit être hydrolysé par une glycoprotéine située à la surface des entérocytes appelée lactase. Cette hydrolyse est indispensable à l'assimilation du lactose, ce dernier ne pouvant être réabsorbé au niveau de l'intestin que sous ses formes glucose et galactose.

Toutefois, une grande partie de la population est déficiente en cette enzyme, soit pour des raisons génétiques, soit parce que son activité diminue avec l'âge, ce qui interdit à ces populations toute consommation de produits riches en lactose.

Or, on a montré que le yaourt, bien que contenant une grande quantité de lactose peut être assimilé par des personnes présentant une déficience en lactase, ce qui laisse supposer que les microorganismes présents dans le yaourt permettaient l'assimilation du lactose.

Le lait étant un aliment de base pour les mammifères (l'homme et les mammifères d'élevage), il est nécessaire d'assurer une bonne assimilation du lactose, constituant important du lait et de certains produits laitiers et ceci tout au long de leur vie ; à cet égard, les biorégulateurs microbiens, à l'exemple de ceux présents dans les laits fermentés peuvent, par leur activité β-galactosidasique, être de précieuses aides à cette assimilation.

Toutefois, dans le cas des produits lactés de l'Art antérieur, contenant des biorégulateurs microbiens, ces produits ont une durée de conservation courte, même à basse température, du fait de l'impossibilité de les stabiliser par traitements thermiques, car de tels traitements détruiraient les biorégulateurs habituellement présents dans le tractus digestif et présentant une activité β-galactosidasique, et rendraient alors ces produits inefficaces dans le tractus digestif.

C'est pourquoi, la Demanderesse s'est donné pour but de pourvoir à un additif alimentaire microbien, comprenant une souche de *Sporolactobacillus*, particulièrement utile comme biorégulateur du lactose, et qui répond mieux aux besoins de la pratique que les additifs de l'Art antérieur, notamment en ce qu'il se présente sous forme de spores naturelles résistantes aux traitements thermiques, et plus particulièrement à la pasteurisation, utilisée de manière habituelle dans les procédés de fabrication de produits lactés ou à la granulation à la vapeur, utilisée dans la préparation des aliments pelletisés, pour l'alimentation animale et en ce qu'il augmente ainsi la durée de conservation des aliments les contenant.

La présente invention a pour objet une souche de *Sporolactobacillus*, caractérisée en ce que l'ADN de ladite bactérie présente notamment des sites de clivage par les endonucléases de restriction Bgl II, Cla I, Sma I, Xba I, Bgl I, Pvu II, EcoR I, Hind III, EcoR V, Xho I, Pst I, Mlu I, lesdites enzymes de restriction fournissant respectivement les fragments suivants (exprimé en Kilobasepaires en Kpb) :

- Bgl II : 20,4 - 14,9 - 8,7 - 7,2.
- Cla I : 9,5 - 9,3 - 7,6 - 6,8 - 6,4 - 4,2 - 3,2 - 2,6.
- Sma I : 20,4 - (16,7) - (14,9) - 13,5 - 10,5 - (8,3) - 7,6 - 5,9 - 4,0.
- Xba I : 20,4 - 19,8 - 18,5 - 10,5 - 8,3 - 6,8 - (4,5) - (2,0).
- Bgl I : (20,4) - 18,5 - (16,7) - 12,6 - 10,7 - 6,8.
- Pvu II : 14,9 - 12,5 - 10,6 - 8,4 - 7,5 - 6,5 - (3,2).
- EcoR I : 14,4 - 10,6 - 5,8 - 5,3 - 3,5 - 2,7 - 2,5.
- Hind III : 17,0 - 7,9 - 7,4 - 7,0 - 5,3 - 3,8 - 3,7 - 3,5 - (3,3) - (3,2) - (2,9) - 1,8.
- EcoR V : (17,0) - 13,2 - 12,0 - 8,1 - 6,3 - 5,3 - (3,5).
- Xho I : 22,8 - 20,6 - 8,5 - 8,1.
- Pst I : 13,2 - 8,5 - 7,8 - 7,3 - 7,0 - 6,6 - (4,1) - (3,2) - (2,5).
- Mlu I : (20,6) - 17,0 - 9,5 - 8,1 - 7,0 - 5,8.

Le genre *Sporolactobacillus* a été plus particulièrement décrit dans BERGEY's Bacteriological Manual (Ed. Lavoisier, 1986, 1139-1141).

La méthode de clivage enzymatique est celle décrite par GRIMONT (Annales de l'Institut Pasteur Microbiologie, 1986, 137B, 165-175).

Cette nouvelle souche, dénommée par les Inventeurs, *Sporolactobacillus* P44, a été déposée auprès de la Collection Nationale de Culture de Microorganismes de l'INSTITUT PASTEUR, en date du 30 avril 1991 sous le n° I-1089.

Cette souche de *Sporolactobacillus* présente notamment des propriétés de biorégulateurs de l'assimilation du lactose non par son activité β-galactosidasique mais par un effet d'induction de la lactase intestinale endogène.

La présente invention a également pour objet un additif alimentaire microbien pour l'alimentation humaine ou animale, caractérisé en ce qu'il comprend au moins une souche de *Sporolactobacillus* présentant des propriétés biorégulatrices, notamment vis-à-vis de l'assimilation du lactose, sélectionnée dans le groupe constitué par la souche *Sporolactobacillus inulinus* et la souche *Sporolactobacillus* P44 conformes à l'invention.

L'espèce *Sporolactobacillus inulinus*, dont la souche de référence est déposée sous le n° ATCC/15538, est plus particulièrement décrite dans le BERGEY Bacteriological Manual (Ed. Lavoisier, 1986, p. 1139-1141).

La Demande de Brevet européen 0 190 770 (DAICEL CHEMICAL INDUSTRIES) décrit un procédé de production d'acide D-lactique mettant en oeuvre une bactérie productrice d'acide D-lactique (*Sporolactobacillus inulinus*).

Or, un additif comprenant une souche de *Sporolactobacillus* telle que définie ci-dessus, présente un certain nombre d'avantages, notamment liés à la présence d'au moins une souche de *Sporolactobacillus* ; en effet, de manière inattendue, l'additif conforme à l'invention :

- résiste aux traitements thermiques, plus particulièrement de pasteurisation et de granulation à la vapeur, notamment du fait de la possibilité de se présenter sous forme de spores naturelles ; et
- présente, aussi bien sous forme d'aliments que d'additifs à destination de l'alimentation humaine qu'animale, des propriétés de biorégulation pour l'assimilation du lactose, par un effet d'induction de la lactose endogène.

Selon un mode de réalisation avantageux de l'additif conforme à l'invention, il comprend au moins la souche de *Sporolactobacillus P44* conforme à l'invention.

Selon encore un autre mode de réalisation avantageux de l'additif conforme à l'invention, il comprend, en outre, en association toute autre souche appropriée agissant en symbiose ou en complément de la/les souches de *Sporolactobacillus*. Un tel additif comprend avantageusement l'association de deux souches appartenant au genre *Sporolactobacillus*.

Conformément à l'invention, l'additif peut, en outre, également être associé à des supports, diluants, conservateurs et autres composants appropriés.

La présente invention a également pour objet des aliments supplémentés aussi bien pour l'alimentation animale qu'humaine, caractérisés en ce qu'ils contiennent l'additif conforme à l'invention.

Ces aliments supplémentés, conformes à l'invention, peuvent être présentés sous toute forme connue en alimentation, forme d'aliment simple ou forme d'aliment composé, etc...

Dans le cadre de l'alimentation animale, les formes d'aliments utilisées seront celles habituellement connues en élevage : poudre, liquide, pâteux ou granulés, etc...

Dans le cadre de l'alimentation humaine, les formes d'aliments utilisées seront celles habituellement connues : poudre, pâte, liquide, etc...

Outre les dispositions qui précèdent, l'invention comprend encore d'autres dispositions, qui ressortiront de la description qui va suivre, qui se réfèrent à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : Caractéristiques morohologiques et biochimiques de la souche *Sporolactobacillus* P44 selon l'invention.

La souche *Sporolactobacillus P44* possède les caractères phénotypiques du genre *Sporolactobacillus* ; elle possède en outre certaines caractéristiques génomiques spécifiques de son espèce.

- Les caractéristiques morphologiques de cette souche sont les suivantes :

  . bacilles droits ou légèrement incurvés, assez fins,
  . extrémités arrondies, de largeur 3 à 5 $\mu$m et de diamètre 0,7 - 0,8 $\mu$m ; ils se présentent sous forme de bacilles isolés ou par paires, rarement sous forme de chaînettes courtes ;
  . mobilité positive par ciliature péritriche ;
  . spores centrales déformantes.

- Les caractéristiques biochimiques sont les suivantes :

  . Gram + ;
  . microaérophile à anaérobie facultatif ;
  . température optimale de croissance : 30 à 37°C ;
  . homo-fermentaire ;
  . absence de catalase et de cytochromes ;
  . pH de fin de culture sur bouillon MRS (après incubation en 48 heures en microaérophilie à 37°C) : environ 4,30 ;
  . dans ces mêmes conditions, forte production d'acide lactique :

    * acide D - lactique : environ 54 %,
    * acide L - lactique : environ 46 %,

  . dans des conditions différentes (culture sur bouillon MRS ensemencé à 5 % et incubé 24 heures à 37°C en aérobiose), il y a production :

    * d'acides gras volatils (99,9 % de $C_2$ et traces de $C_4$),
    * et d'acide lactique :

      Isomère D : 97,5 %
      Isomère L : 2,5 %.

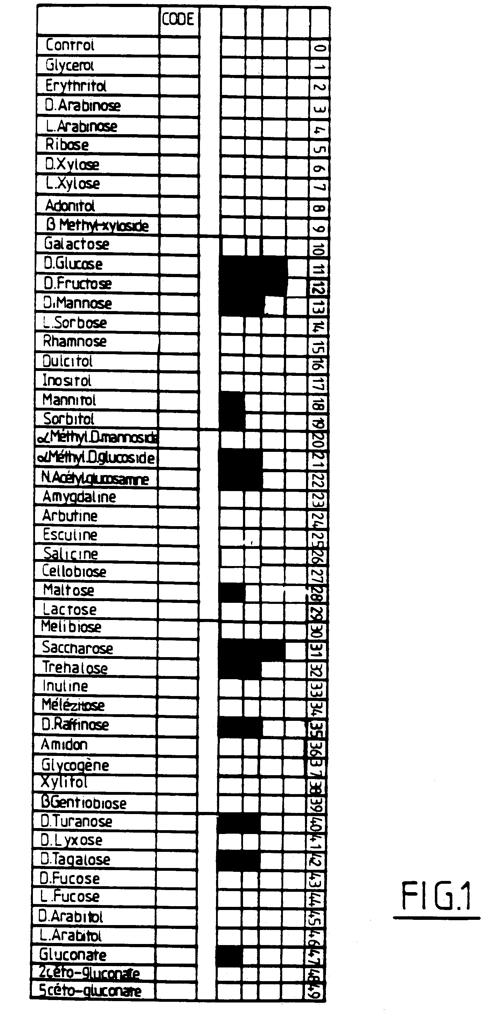

Les résultats d'une galerie API 50 CHL donnant le profil de fermentation des sucres sont présentés sur la figure 1 ; la souche est inoculée selon les spécifications API 50 CHL avec une suspension de densité optique d'environ 0,5 à 0,75 mesurée à 550 nm, soit à tube 5 dans l'échelle de Mac Farland.

### EXEMPLE 2 : Profils de restriction comparés de *Sporolactobacillus P44 et de Sporolactobacillus inulinus.*

a) <u>Protocole opératoire</u> :

La méthodologie utilisée est celle décrite par GRIMONT (Annales Institut Pasteur Microbiologie, 1986, 137 B, 165-175) et comprend les étapes suivantes :

- lyse des bactéries et extraction de l'ADN chromosomique,
- dénaturation de l'ADN par traitement au phénol et au chloroforme et purificaticn par précipitation à l'éthanol,
- clivage de l'ADN avec 25 endonucléases de restriction et séparation des fragments obtenus par électrophorèse en agarose,
- transfert de Southern des fragments d'ADN obtenus, sur membrane de nylon (transfert de Southern modifié, en utilisant le transfert alcalin sous vide),
- hybridation des fragments avec de l'ARNr 16 et 23 S d'*Escherichia coli*, marqué à l'extrémité 5' par du phosphore radioactif,
- lavage et visualisation par autoradiographie des fragments d'ADN portant les gènes codant pour les acides ribonucléiques ribosomaux ARN (ARNr).

b) <u>Profil de *Sporolactobacillus P44*</u> :

12 endonucléases ont été capables de cliver l'ADN de cette souche : Bgl II, Cla I, Sma I, Xba I, Bal I, Pvu II, EcoR I, Hind III, EcoR V, Xho I, Pst I, Mlu I ; le Tableau I ci-après donne les poids moléculaires des fragments de restriction d'ADN du *Sporolactobacillus* P44, hybridant avec l'ARN 16 + 23S d'*Escherichia coli*.

TABLEAU I

- Bgl II : 20,4 - 14,9 - 8,7 - 7,2.

- Cla I : 9,5 - 9,3 - 7,6 - 6,8 - 6,4 - 4,2 - 3,2 - 2,6.

- Sma I : 20,4 - (16,7) - (14,9) - 13,5 - 10,5 - (8,3) -7,6 - 5,9 - 4,0.

- Xba I : 20,4 - 19,8 - 18,5 - 10,5 - 8,3 - 6,8 - (4,5) -(2,0).

- Bgl I : (20,4) - 18,5 - (16,7) - 12,6 - 10,7 - 6,8.

- Pvu II : 14,9 - 12,5 - 10,6 - 8,4 - 7,5 - 6,5 - (3,2).

- EcoR I : 14,4 - 10,6 - 5,8 - 5,3 - 3,5 - 2,7 - 2,5.

- Hind III : 17,0 - 7,9 - 7,4 - 7,0 - 5,3 - 3,8 - 3,7 -3,5 - (3,3) - (3,2) - (2,9) - 1,8.

- EcoR V : (17,0) - 13,2 - 12,0 - 8,1 - 6,3 - 5,3 -(3,5).

- Xho I : 22,8 - 20,6 - 8,5 - 8,1.

- Pst I : 13,2 - 8,5 - 7,8 - 7,3 - 7,0 - 6,6 - (4,1) -(3,2) - (2,5).

- Mlu I : (20,6) - 17,0 - 9,5 - 8,1 - 7,0 - 5,8.

c) <u>*Profil de Sporolactobacillus inulinus*</u> :

Dans les mêmes conditions que celles exposées en a) et b) ci-dessus, on obtient pour *Sporolactobacillus inulinus*, le profil illustré au Tableau II, ci-après :

TABLEAU II

- Bgl II : 14,9 - 13,5 - 10,6 - 9,4 - 8,7 - 6,8

- Cla I : 16,7 - 13,5 - 12,5 - (9,3) - 8,3 - 7,2

- Sma I : 20,4 - 16,7 - 12,5 - (11,6) - 8,7 - 6,5 - 3,2 -2,0

- Xba I : 20,4 - (18,5) - 8,3 - 7,2 - (4,5)

TABLEAU II (suite)

- Bgl I : (20,4) - 18,5 - 12,5 - 10,5 - 7,2

- Pvu II : 13,5 - 10,4 - 9,7 - 7,6 - (4,5) - (3,2)

- EcoR I : 22,8 - 20,6 - 17,0 - 14,4 - 12,0 - 9,5 - 8,5 -4,8 - (2,5)

- Hind III : 17,0 - 9,5 - 9,0 - 6,2 - 4,1 - 3,8 - 3,7 -3,5 - 3,2 - (1,8)

- EcoR V : 13,2 - 10,6 - 7,9 - 6,3 - 6,2 - (5,3) - (3,5)

- Xho I : 22,8 - 20,6 - 14,4 - 12,0 - 8,1

- Pst I : 8,5 - 7,4 - 5,8 - (4,1) - (3,2) - (2,5)

- Mlu I : 22,8 - 17,0 - 14,4 - 8,1 - 7,0

Dans ces deux Tableaux, les bandes faibles, nécessitant une surexposition des films pour être observables, sont précisées entre parenthèses.

d) Etude comparative des résultats illustrés aux Tableaux I et II :

Les profils illustrés aux Tableaux I et II montrent que ces deux souches sont nettement différentes.

**EXEMPLE 3 : Effet biorégulateur du *Sporolactobacillus P44* sur l'assimilation du lactose selon l'invention par rapport à un lot témoin et à une souche *Clostridium butyricum* 1002.5, sur des rats mâles de race Fisher 344.**

Les essais sont réalisés sur trois lots de rats gnotoxéniques élevés dans des isolateurs expérimentaux stériles selon un dispositif expérimental décrit par LECOZ et al. (Sci. Techn. Anim. Lab., (1989), 14, (1), p. 35-39).
Les aliments dont ils sont nourris sont stérilisés ; ce sont des régimes semi-synthétiques adaptés au rat à base d'amidon de mais et de farine de poisson dont une fraction de l'amidon a été remplacée soit par 8 % de lactose (Merck) (régime lactose 8 %), soit par 2 % de lactulose (Duphalac, Duphar) (régime lactulose 2 %).
Ces trois lots sont conformes à la description suivante :

1. rats axéniques (lot témoin).
2. rats à l'état monoxénique (avec apport d'une seule souche microbienne dans l'alimentation) :

\*    soit avec la souche de *Clostridium butyricum* 1002.5 décrite dans POPOFF et al. (Infection and Immunity (1985), 47, 3, p. 697-776).

Les rats sont assoiffés durant une nuit et reçoivent le lendemain matin comme eau de boisson, 5 ml d'un bouillon de culture de LAPTTWISS (10 g autolysat de levure DIFCO, 15 g peptone Evans, 10 g tryptone DIFCO, 1 g Tween 80, 1 l d'eau distillée, pH = 6,5), de 24 heures ($10^8$ *C. butyricum*/ml), dilué dans environ 20 ml d'eau distillée.
Après consommation de "cette solution", les rats reçoivent de nouveau de l'eau distillée comme boisson.

\*    soit au *Sporolactobacillus P44* : en fonction des besoins, la suspension bactérienne ($10^7$ germes/ml) est diluée au quart dans de l'eau distillée stérile et versée dans les biberons. La souche devant être donnée en continu, un nouvel aliquot est introduit dans l'isolateur toutes les 48 heures.

3. rats à l'état dixénique (avec apport continu de *Sporolactobacillus P44* sur rats monoxéniques après implantation dans l'intestin de *Clostridium butyricum* 1002.5) :
Les rats monoxénisés au *C. butyricum* 1002.5 reçoivent en continu le *Sporolactobacillus P44* dans leur eau de boisson, comme décrit ci-dessus.
Le rat a été choisi, car il possède un équipement enzymatique intestinal endogène proche de celui des jeunes mammifères.

On a mesuré sur ces différents lots les paramètres métaboliques suivants :

\*    Caractéristiques de l'assimilation du lactose et/ou du lactulose ingérés,

- mesure de l'hydrogène excrété,
- bilan lactose/lactulose : détermination de la quantité de lactose ou de lactulose ingérée et excrétée quotidiennement afin d'en déduire la quantité de sucre métabolisée par l'animal, ce qui permet de calculer le CUD (Coefficient d'Utilisation Digestive) de chacun de ces sucres.

$$CUD = \frac{\text{quantité sucre ingérée-quantité sucre excrétée x 100}}{\text{quantité sucre ingérée}}$$

- dosage des activités spécifiques de la β-galactosidase dans les fécès et de la lactase neutre endogène de la muqueuse intestinale.

\* Caractéristiques fermentaires des 2 souches utilisées :

- *Sporolactobacillus P44* :

Les produits de fermentation présents dans le milieu de culture MRS dans les conditions de l'exemple 1 sont :

. des acides gras volatils : acétate 99,8 % et butyrate (traces),
. de l'acide lactique : plus grande quantité d'isomère D (97,5 %) que L (2,5 %).

Cette souche ne présente pas de β-galactosidase constitutive ni inductible.

- *Clostridium butyricum* 1002.5 :

Cette souche produit :

. des AGV : beaucoup plus de butyrate (63 %) que d'acétate (37 %),
. de l'acide lactique : faible quantité,
. des gaz (hydrogène).

Cette souche possède une β-galactosidase inductible avec le lactose, dont l'activité est maximale à 40°C (pH = 7).
Ces caractéristiques sont résumées dans le Tableau III ci-après :

TABLEAU III

| Bactéries | | Bacille sporulé P44 | C. butyricum (BOUSSEBOUA. 1989) | |
|---|---|---|---|---|
| Métabolites fermentaires | | Milieu MRS | glucose | lactose |
| | $c_T$ | 101,2 | 34,1 | 36 |
| AGV (mmoles/l) | $c_2$ (%) | 101 100 | 12,6 37 | 13,3 36,9 |
| | $c_4$ (%) | 0,2 0,2 | 21,5 63 | 22,7 63,1 |
| | $c_T$ | 113,4 | | 1,2 |
| Acide lactique (mmole/l) | D (%) | 110,6 97,5 | | |
| | L (%) | 2,8 2,5 | | |
| Gaz (hydrogène) | | - | + | + |
| β-galactosidase | | - | + | |

RESULTATS :

Dans les régimes lactose 8 % et lactulose 2 %, la fermentation du lactose comme celle du lactulose se traduit par une production d'hydrogène chez les rats monoxéniques en *Clostridium butyricum* 1002.5.

Quand on apporte à ces rats en continu la souche du *Sporolactobacillus P44*, on obtient une diminution de la production de gaz uniquement chez les rats en régime lactose 8 %, cette diminution étant due à une plus faible quantité de sucre fermenté par le *Clostridium butyricum* 1002.5.

Les rats axéniques et monoxéniques avec la souche *Sporolactobacillus P44*, ingérant la même quantité de lactose, l'excrètent dans des proportions différentes : le CUD du lactose des rats monoxéniques qui est égal à (95,2 ± 0,4) est significativement plus élevé que le CUD des rats axéniques (91,7 ± 0,6), ce qui indique que la quantité de lactose métabolisée chez les animaux monoxéniques avec cette souche, est améliorée de 3,5 %.

De plus, les rats dixéniques dégradent totalement le lactose et le lactulose (CUD = 100 %), du fait de leur fermentation dans le gros intestin par *Clostridium butyricum* 1002.5.

Les résultats de la mesure de l'activité spécifique de la lactase à différents niveaux de l'intestin indiquent que l'apport continu de la souche *Sporolactobacillus P44* induit chez le rat monoxénique une augmentation de l'activité spécifique lactasique duodénale.

Ces résultats mettent parfaitement en évidence le rôle biorégulateur de la souche *Sporolactobacillus P44* dans l'assimilation du lactose et donc toute l'importance de l'utilisation de cette souche dans des produits laitiers riches en lactose destinés à des populations plus particulièrement déficientes en lactase.

En outre, du fait de la possibilité de présenter la souche sous forme de spores naturelles, cette souche peut être utilisée dans des aliments nécessitant des traitements thermiques et/ou mécaniques importants tout en gardant ses propriétés biorégulatrices.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Souche de *Sporolactobacillus*, caractérisée en ce que l'ADN de ladite bactérie présente notamment des sites de clivage par les endonucléases de restriction Bgl II, Cla I, Sma I, Xba I, Bgl I, Pvu II, EcoR I, Hind III, EcoR V, Xho I, Pst I, Mlu I, lesdites enzymes de restriction fournissant respectivement les fragments suivants (exprimé en Kilobasepaires en Kpb) :

   - Bgl II : 20,4 - 14,9 - 8,7 - 7,2.
   - Cla I : 9,5 - 9,3 - 7,6 - 6,8 - 6,4 - 4,2 - 3,2 - 2,6.
   - Sma I : 20,4 - (16,7) - (14,9) - 13,5 - 10,5 - (8,3) - 7,6 - 5,9 - 4,0.
   - Xba I : 20,4 - 19,8 - 18,5 - 10,5 - 8,3 - 6,8 - (4,5) - (2,0).
   - Bgl I : (20,4) - 18,5 - (16,7) - 12,6 - 10,7 - 6,8.
   - Pvu II : 14,9 - 12,5 - 10,6 - 8,4 - 7,5 - 6,5 - (3,2).
   - EcoR I : 14,4 - 10,6 - 5,8 - 5,3 - 3,5 - 2,7 - 2,5.
   - Hind III : 17,0 - 7,9 - 7,4 - 7,0 - 5,3 - 3,8 - 3,7 - 3,5 - (3,3) - (3,2) - (2,9) - 1,8.
   - EcoR V : (17,0) - 13,2 - 12,0 - 8,1 - 6,3 - 5,3 - (3,5).
   - Xho I : 22,8 - 20,6 - 8,5 - 8,1.
   - Pst I : 13,2 - 8,5 - 7,8 - 7,3 - 7,0 - 6,6 - (4,1) - (3,2) - (2,5).
   - Mlu I : (20,6) - 17,0 - 9,5 - 8,1 - 7,0 - 5,8.

2. Souche de *Sporolactobacillus* P44 selon la revendication 1, caractérisée en ce qu'elle a été déposée auprès de la Collection Nationale de Culture de Microorganismes de l'INSTITUT PASTEUR, en date du 30 avril 1991 sous le n° I-1089.

3. Additif alimentaire microbien pour l'alimentation humaine ou animale, caractérisé en ce qu'il comprend au moins une souche de *Sporolactobacillus* P44 présentant des propriétés biorégulatrices, notamment vis-à-vis de l'assimilation du lactose selon la revendication 1 ou la revendication 2.

4. Additif alimentaire microbien selon la revendication 3, caractérisé en ce qu'il comprend une autre souche de *Sporolactobacillus*.

5. Additif alimentaire microbien selon l'une quelconque des revendications 3 ou 4, caractérisé en ce qu'il peut, en outre, également être associé à des supports, diluants, conservateurs et autres composants appropriés.

6. Aliments supplémentés aussi bien pour l'alimentation animale qu'humaine, caractérisés en ce qu'ils contiennent l'additif selon l'une quelconque des revendications 3 à 5.

## Claims

1. Strain of *Sporolactobacillus,* characterised in that the DNA of the said bacterium presents, in particular, the restriction endonuclease cleavage sites Bgl II, Cla I, Sma I, Xba I, Bgl I, Pvu II, EcoR I, Hind III, EcoR V, Xho I, Pst I, Mlu I, the said restriction enzymes respectively providing the following fragments (expressed in kilobasepairs in Kbp) :

   - Bgl II : 20.4 - 14.9 - 8.7 - 7.2.
   - Cla I : 9.5 - 9.3 - 7.6 - 6.8 - 6.4 - 4.2 - 3.2 - 2.6.
   - Sma I : 20.4 - (16.7) - (14.9) - 13.5 - 10.5 - (8.3) - 7.6 - 5.9 - 4.0.
   - Xba I : 20.4 - 19.8 - 18.5 - 10.5 - 8.3 - 6.8 - (4.5) - (2.0).
   - Bgl I : (20.4) - 18.5 - (16.7) - 12.6 - 10.7 - 6.8.
   - Pvu II : 14.9 - 12.5 - 10.6 - 8.4 - 7.5 - 6.5 - (3.2).
   - EcoR I : 14.4 - 10.6 - 5.8 - 5.3 - 3.5 - 2.7 - 2.5.
   - Hind III : 17.0 - 7.9 - 7.4 - 7.0 - 5.3 - 3.8 - 3.7 - 3.5 - (3.3) - (3.2) - (2.9) - 1.8.
   - EcoR V : (17.0) - 13.2 - 12.0 - 8.1 - 6.3 - 5.3 - (3.5).
   - Xho I : 22.8 - 20.6 - 8.5 - 8.1.
   - Pst I : 13.2 - 8.5 - 7.8 - 7.3 - 7.0 - 6.6 - (4.1) - (3.2) - (2.5).
   - Mlu I : (20.6) - 17.0 - 9.5 - 8.1 - 7.0 - 5.8.

2. Strain of *Sporolactobacillus* P44 according to claim 1, characterised in that it has been filed with the Collection Nationale de Culture de Micro-organismes of the INSTITUT PASTEUR on 30 April 1991 under the n° I-1089.

3. Microbial food additive for human or animal food, characterised in that it comprises at least one strain of *Sporolactobacillus* P44 presenting bioregulatory properties, in particular with respect to the assimilation of lactose according to claim 1 or claim 2.

4. Microbial food additive according to claim 3, characterised in that it comprises another strain of *Sporolactobacillus*.

5. Microbial food additive according to any one of claims 3 or 4, characterised in that it may, furthermore, also be associated with supports, diluents, preserving agents and other appropriate compounds.

6. Food supplements as well for animal food as for human, characterised in that they contain the additive according to any one of claims 3 to 5.

**Patentansprüche**

1. *Sporolactobacillus*-Stamm, dadurch gekennzeichnet, daß die DNA des Bakteriums insbesondere Spaltungsstellen für die Restriktions-Endonucleasen Bgl II, Cla I, Sma I, Xba I, Bgl I, Pvu II, EcoR I, Hind III, EcoR V, Xho I, Pst I, Mlu I aufweist, wobei die Restriktionsenzyme jeweils die folgenden Fragmente liefern (ausgedrückt als Kilobasenpaare in Kbp):

- Bgl II: 20,4 - 14,9 - 8,7 - 7,2.
- Cla I: 9,5 - 9,3 - 7,6 - 6,8 - 6,4 - 4,2 - 3,2 - 2,6.
- Sma I: 20,4 - (16,7) - (14,9) - 13,5 - 10,5 - (8,3) - 7,6 - 5,9 - 4,0.
- Xba I: 20,4 - 19,8 - 18,5 - 10,5 - 8,3 - 6,8 - (4,5) - (2,0).
- Bgl I: (20,4) - 18,5 - (16,7) - 12,6 - 10,7 - 6,8.
- Pvu II: 14,9 - 12,5 - 10,6 - 8,4 - 7,5 - 6,5 - (3,2).
- EcoR I: 14,4 - 10,6 - 5,8 - 5,3 - 3,5 - 2,7 - 2,5.
- Hind III: 17,0 - 7,9 - 7,4 - 7,0 - 5,3 - 3,8 - 3,7 - 3,5 - (3,3) - (3,2) - (2,9) - 1,8.
- EcoR V: (17,0) - 13,2 - 12,0 - 8,1 - 6,3 - 5,3 - (3,5).
- Xho I: 22,8 - 20,6 - 8,5 - 8,1.
- Pst I: 13,2 - 8,5 - 7,8 - 7,3 - 7,0 - 6,6 - (4,1) - (3,2) - (2,5).
- Mlu I: (20,6) - 17,0 - 9,5 - 8,1 - 7,0 - 5,8.

2. *Sporolactobacillus*-Stamm P44 nach Anspruch 1, dadurch gekennzeichnet, daß er am 30.04.1991 unter der Nummer I-1089 bei der Collection Nationale de Culture de Microorganismes des INSTITUT PASTEUR hinterlegt wurde.

3. Mikrobieller Lebensmittelzusatz für die menschliche oder tierische Ernährung, dadurch gekennzeichnet, daß er zumindest einen *Sporolactobacillus*-Stamm P44 nach Anspruch 1 oder 2 mit physiologisch regulierenden Eigenschaften, insbesondere im Hinblick auf die Assimilierung von Lactose, aufweist.

4. Mikrobieller Lebensmittelzusatz nach Anspruch 3, dadurch gekennzeichnet, daß er einen weiteren *Sporolactobacillus*-Stamm aufweist.

5. Mikrobieller Lebensmittelzusatz nach einem der Ansprüche 3 oder 4, dadurch gekennzeichnet, daß er des weiteren auch mit Trägern, Verdünnungsmitteln, Konservierungsmitteln und weiteren geeigneten Inhaltsstoffen kombiniert sein kann.

6. Nahrungsmittelergänzungsstoffe für die tierische wie auch menschliche Ernährung, dadurch gekennzeichnet, daß sie den Zusatz gemäß einem der Ansprüche 3 bis 5 enthalten.

| | CODE | | | | | | | |
|---|---|---|---|---|---|---|---|---|
| Control | | | | | | | | 0 |
| Glycerol | | | | | | | | 1 |
| Erythritol | | | | | | | | 2 |
| D.Arabinose | | | | | | | | 3 |
| L.Arabinose | | | | | | | | 4 |
| Ribose | | | | | | | | 5 |
| D.Xylose | | | | | | | | 6 |
| L.Xylose | | | | | | | | 7 |
| Adonitol | | | | | | | | 8 |
| β Methyl-xyloside | | | | | | | | 9 |
| Galactose | | | | | | | | 10 |
| D.Glucose | | | | | | | | 11 |
| D.Fructose | | | | | | | | 12 |
| D.Mannose | | | | | | | | 13 |
| L.Sorbose | | | | | | | | 14 |
| Rhamnose | | | | | | | | 15 |
| Dulcitol | | | | | | | | 16 |
| Inositol | | | | | | | | 17 |
| Mannitol | | | | | | | | 18 |
| Sorbitol | | | | | | | | 19 |
| α Méthyl.D.mannoside | | | | | | | | 20 |
| α Méthyl.D.glucoside | | | | | | | | 21 |
| N.Acétyl.glucosamine | | | | | | | | 22 |
| Amygdaline | | | | | | | | 23 |
| Arbutine | | | | | | | | 24 |
| Esculine | | | | | | | | 25 |
| Salicine | | | | | | | | 26 |
| Cellobiose | | | | | | | | 27 |
| Maltose | | | | | | | | 28 |
| Lactose | | | | | | | | 29 |
| Melibiose | | | | | | | | 30 |
| Saccharose | | | | | | | | 31 |
| Trehalose | | | | | | | | 32 |
| Inuline | | | | | | | | 33 |
| Mélézitose | | | | | | | | 34 |
| D.Raffinose | | | | | | | | 35 |
| Amidon | | | | | | | | 36 |
| Glycogène | | | | | | | | 37 |
| Xylitol | | | | | | | | 38 |
| βGentiobiose | | | | | | | | 39 |
| D.Turanose | | | | | | | | 40 |
| D.Lyxose | | | | | | | | 41 |
| D.Tagatose | | | | | | | | 42 |
| D.Fucose | | | | | | | | 43 |
| L.Fucose | | | | | | | | 44 |
| D.Arabitol | | | | | | | | 45 |
| L.Arabitol | | | | | | | | 46 |
| Gluconate | | | | | | | | 47 |
| 2céto-gluconate | | | | | | | | 48 |
| 5céto-gluconate | | | | | | | | 49 |

FIG.1